# EUROPÄISCHE PATENTSCHRIFT

(11) **EP 1 439 737 B1**
(45) Veröffentlichungstag und Bekanntmachung des Hinweises auf die Patenterteilung: **13.03.2013**
(21) Anmeldenummer: 04450006.4
(22) Anmeldetag: 14.01.2004
(51) Int. Cl.: A61F 2/18

(54) **Implantierbarer elektromechanischer Wandler**
Implantable electromechanical transducer
Transducteur électromécanique implantable

(30) Priorität: 15.01.2003 DE 10301723
(43) Veröffentlichungstag der Anmeldung: 21.07.2004
(73) Patentinhaber: Med-El Elektromedizinische Geräte Ges.m.b.H, 6020 Innsbruck (AT)
(72) Erfinder: Blau, Matthias, 26188 Edewecht (DE); Hofmann, Gert, 01465 Langebrück (DE); Bornitz, Matthias, 01157 Dresden (DE); Hüttenbrink, Karl-Bernd, 01326 Dresden (DE); Zahnert, Thomas, 01326 Dresden (DE)
(74) Vertreter: Gibler, Ferdinand

(56) Entgegenhaltungen:
- EP-A- 1 011 295
- WO-A-97/11575
- US-A- 4 729 366
- US-A- 5 842 967

## Beschreibung

Die Erfindung betrifft einen implantierbaren elektromechanischen Wandler für Cochlea-Implantate und implantierbare Hörgeräte gemäß dem Oberbegriff des Anspruchs 1.

Vollständig oder teilweise implantierbare Hörhilfen wie Cochlea-Implantate oder implantierbare Hörgeräte benötigen einen implantierbaren Wandler zum Aufnehmen des am Ohr des Patienten eintreffenden Schalls, welcher in elektrische Signale für die weitere Verarbeitung in der entsprechenden Hörhilfe gewandelt werden muss.

Zu diesem Zweck sind verschiedene Lösungsansätze vorgeschlagen worden. Ein erster Ansatz stellt die direkte Umwandlung des am Ohr eintreffenden Luftschalls in elektrische Signale dar, welche mit verschiedenen Mitteln realisiert werden kann, die aus der US 3 882 285, US 4 988 333, WO 96/21333, US 5 411 467, EP 0 831 673 bekannt sind. Nachteile dieses Ansatzes sind, dass meist nicht die natürliche Richtungsfilterung des Außenohres benutzt wird und/oder dass die Fixation/Implantierung der benötigten Wandlerkomponenten zu adversen Reaktionen des betroffenen/umgebenden Gewebes führen kann.

In einem zweiten Ansatz wird der natürliche Schallempfangsmechanismus des menschlichen Außen- und Mittelohres zur Umwandlung des eintreffenden Schalls in Schwingungen der Mittelohrkomponenten (Trommelfell und Gehörknöchelchen) benutzt, um dann diese Schwingungen in elektrische Signale umzuwandeln. Hierfür sind unterschiedliche Wandlerprinzipien vorgeschlagen worden: Aus der US 3 870 832 und der US 5 554 096 sind implantierbare Wandler auf der Grundlage des elektromagnetischen Prinzips bekannt. Der relativ hohe Energieverbrauch elektromagnetischer und elektrodynamischer Wandler schränkt jedoch ihre Anwendbarkeit für implantierbare Cochlea-Implantate und Hörgeräte ein.

Dieser Nachteil wird durch Wandler auf der Grundlage des piezoelektrischen Prinzips vermieden. Aus der EP 0 263 254 ist ein implantierbarer Wandler aus Piezofilm, Piezokristall oder einem piezoelektrischen Beschleunigungsaufnehmer bekannt, bei dem ein Ende im Knochen einzementiert ist und das andere Ende mit einer schwingenden Komponente des Mittelohres starr verbunden ist. Dabei besteht jedoch das Problem, dass starre Verbindungen zu den Gehörknöchelchen die Gefahr der Knochenarrosion bergen und dass eine Einzementierung von Wandlerkomponenten im Mittelohrraum auf mechanische und toxikologische Vorbehalte stößt. Außerdem werden keine Angaben gemacht, auf welche Weise ein Kontakt von Piezomaterialien und Körperflüssigkeit dauerhaft verhindert werden soll, so dass zum einen mit Blokompatibilitätsproblemen und zum anderen mit einer Einbuße der piezoelektrischen Eigenschaften durch physikalische und chemische Interaktionen von Piezomaterial und Körperflüssigkeit gerechnet werden muss.

Aus der US 3 712 962 ist ein implantierbarer Wandler bekannt, bei dem ein piezoelektrischer Zylinder oder ein piezoelektrischer Biegebalken als Wandlerkomponente vorgesehen sind, wobei wiederum ein nicht näher spezifizierter Ankerpunkt im Ohr vorgesehen ist. Ähnlich wie bei der oben zitierten EP 0 263 254 werden auch hier keine Angaben gemacht, auf welche Weise ein Kontakt von Piezomaterialien und Körperflüssigkeit dauerhaft verhindert werden soll.

Aus der WO 99/08480 ist ein implantierbarer Wandler auf der Grundlage des piezoelektrischen Prinzips bekannt, der ausschließlich an einer schwingenden Mittelohrkomponente fixiert ist, und bei dem das Gegenlager durch eine mit dem Wandler verbundene Inertialmasse gebildet wird. Problematisch hierbei ist jedoch, dass die Fixierung an eine schwingende Mittelohrkomponente wie z.B. das Trommelfell oder die Gehörknöchelchen entweder mechanisch nicht dauerhaft stabil gestaltet werden kann oder die Gefahr der Knochenarrosion birgt, welche aufgrund der im Vergleich zu passiven Mittelohrimplantaten höheren Masse des implantierbaren Wandlers ein höheres Risiko darstellt als bei diesen.

Aus der WO 94/17645 ist ein implantierbarer Wandler auf der Grundlage des kapazitiven bzw. des piezoresistiven Prinzips bekannt, welcher mittels mikromechanischer Technologien hergestellt werden soll. Dieser Wandler soll als Druckempfänger im Amboß-Steigbügel-Gelenk arbeiten. Da der Steigbügel mit dem angekoppelten Innenohr ein Resonanzsystem bildet, ist jedoch zu befürchten, dass keine ausreichende Empfindlichkeit im gesamten Nutzfrequenzbereich erreicht werden kann. Diese Befürchtung gilt auch für die auf hydroakustischer Signalübertragung beruhenden implantierbaren Wandler nach WO 97/18689 und DE 100 30 372.

Die US 5 842 967 A beschreibt einen piezoelektrischen Wandler, welcher in einem biokompatiblen Gehäuse im Bereich der Mittelohrknochen angeordnet werden kann, wobei zur Übertragung der Schwingungen von einem der Gehörknochen auf den Wandler eine Anordnung aus sich abstoßenden Permanentmagneten vorgesehen ist.

Die WO 97/11575 A beschreibt eine Hörgeräteanordnung mit einem Mikrofon und einem Mikroaktuator.

Die EP 1 011 295 A beschreibt einen elektroakustischen Wandler, welcher in der Ausführungsform gemäß Fig. 2 ein hermetisch dichtes Gehäuse mit einer biegeweichen Fläche aufweist, an deren Innenseite ein piezoelektrischer Wandler angeordnet ist.

Aus dem Stand der Technik lässt sich die Aufgabe ableiten, ein implantierbares Mikrofon zur Wandlung des am Ohr eintreffenden Schalls in elektrische Signale derart zu gestalten, dass eine ausreichende Empfindlichkeit im gesamten Nutzfrequenzbereich gewährleistet ist, dass möglichst wenig Zusatzenergie benötigt wird, dass die empfindlichen Knochen- und Gewebsstrukturen des Ohres durch eine geeignete Positionierung und Ankoppelung des implantierbaren Mikrofons so wenig wie möglich beansprucht werden und dass die Langzeitstabilität und Biokompatibilität des implantierbaren Mikrofons durch eine hermetisch dichte, kompatible Umhüllung gewährleistet ist.

Die Aufgabe wird in Verbindung mit den im Oberbegriff des Anspruchs 1 genannten Merkmalen dadurch gelöst, dass an der Innenseite des Hohlkörpers die piezoelektrischen Wandlerelement mit der dünnen Schale verbunden sind, und dass die Außenseite der dünnen Schale derart zur flächigen Ankoppelung an ein Gehörknöchelchen vorgesehen ist, dass die Schwingungen der Gehörknöchelchen zu Verformungen der piezoelektischen Wandlcrelemente und zu einer elektrischen Spannung an Elektroden der Wandlerelemente führen.

Erfingdungsgemäß wird die Wandlung von mechanischen Schwingungen in eine elektrische Spannung durch ein oder mehrere piezoelektrische Wandlerelemente realisiert, welche zur Isolation gegen Körperflüssigkeit von einem hermetisch dichten Hohlkörper aus biokompatiblen Material, vorzugsweise aus Titan oder aus einer Titanlegierung, umgeben sind. Dabei ist der Hohlkörper so beschaffen, dass er eine dünne Schale aufweist, die durch einen stabilen Rand gehalten wird, welcher an ein Gegenlager im Mittelohraum ankoppelbar ist.

Die piezoelektrischen Wandlerelemente sind an der Innenseite des Hohlkörpers mit der dünnen Schale verbunden, während die Außenseite der dünnen Schale an ein Gehörknöchelchen angekoppelt wird, so dass die Schwingungen der Gehörknöchelchen zu Verformungen der piezoelektrischen Wandlerelemente und somit zu einer elektrischen Spannung an den Elektroden der Wandlerelemente führen.

Durch die flächige Ankopplung an eines der Gehörknöchelchen werden Beeinträchtigungen der Knochenstrukturen weitestgehend vermieden.
Eine vorteilhafte Ausführung des Wandlers wird beispielsweise erreicht, indem der Hohlkörper als elliptische Dose ausgeformt wird, wobei ein piezoelektrisches Wandlerelement in Form einer dünnen Biegeplatte mit der als dünne elliptische Platte ausgeformten dünnen Schale verbunden ist. In diesem Fall ist es möglich, die Höhe der Dose auf weniger als 1 mm zu reduzieren.

Die Verbindung zwischen stabilem Rand und dünner Schale kann einerseits durch verschiedene Verbindungsverfahren, wie z.B. Schweißen, erfolgen. Andererseits ist es auch möglich, stabilen Rand und dünne Schale aus einem Ganzen durch mechanische Trenn- und Umformverfahren oder durch chemische Verfahren wie z.B. Ätzen herzustellen.

Die Verbindung zwischen piezoelektrischen Wandlerelementen und dünner Schale kann durch Kleben oder durch mechanische Mittel erfolgen. Die piezoelektrischen Wandlerelemente selbst können aus allen bekannten piezoelektrischen Materialien bestehen. Günstig ist jedoch die Verwendung speziell gezüchteter Einkristalle aus Bleimagnesiumniobat-Bleititanat (PMN-PT) oder Bleizinkniobat-Bleititanat (PZN-PT), weil durch deren elektromechanische Eigenschaften die Größe der piezoelektrischen Wandlerelemente und damit die des gesamten Wandlers minimiert werden kann.

Um eine möglichst störungsunanfällige elektrische Signalspannung an den äußeren elektrischen Anschlüssen zur Verfügung zu stellen, sollte bereits im Hohlkörper eine elektronische Schaltung zur Signalkonditionierung vorgesehen werden.

Für die Ausbildung des Gegenlagers gibt es verschiedene Möglichkeiten. Eine Ausführung besteht aus einem in die Nische des ovalen Fensters gestellten Ständer aus biokompatiblen Material, bevorzugt aus Titan oder einer Titanlegierung, welcher den Hohlkörper aufnehmen kann. Eine alternative Möglichkeit ist die Verwendung eines mit einem Knochen des Mittelohrraumes verschraubten Stützelements. In beiden Fällen kann man auf den Einsatz von Klebern verzichten, wodurch das Risiko toxischer Schäden ausgeschlossen ist.

Die Erfindung wird nachfolgend anhand von Ausführungsbeispielen näher erläutert. In den Zeichnungen zeigen:
- Fig. 1: eine Übersicht über die relevanten Strukturen des Mittelohrraums
- Fig. 2: einen Querschnitt des Mittelohrbereiches mit einer Ausführungsform des implantierten Wandlers
- Fig. 3: eine Ausführungsform des Wandlers mit Positionierung durch unterlegbare Plättchen
- Fig. 4: eine Ausfühnmgsform des Hohlkörpers mit geklebten piezoelektrischen Wandlerelementen
- Fig. 5: eine Ausführungsform des Hohlkörpers mit mechanisch gelagerten piezoelektrischen Wandlerelementen
- Fig. 6: eine Ausführungsform des Hohlkörpers mit elektronischer Schaltung zur Sinalkonditionierung
- Fig. 7: eine Ausführungsform des Wandlers mit rastbarer Zweiteilung des Ständers
- Fig. 8: einen Querschnitt des Mittelohrbereiches mit einer weiteren Ausfübrungsform des implantierten Wandlers.

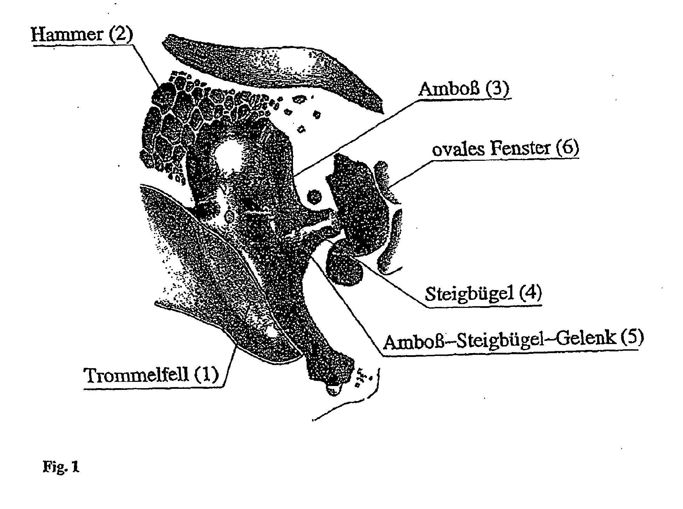

In der Fig. 1 ist eine Übersicht über die relevanten Strukturen des Mittelohrraums gezeigt. Schallwellen erreichen das Trommelfell **1** und versetzen es in Schwingungen, die durch die Gehörknöchelchen Hammer **2**, Amboß **3** und Steigbügel **4** zum ovalen Fenster **6** des Innenohres weitergeleitet werden. Ebenfalls gezeigt ist das Amboß-Steigbügel-Gelenk **5**.

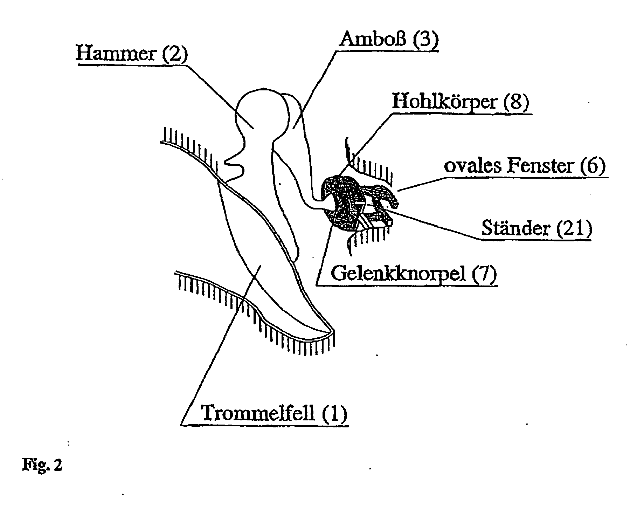

In der Fig. 2 ist ein Querschnitt des Mittelohrbereiches mit einer Ausführungsform des implantierten Wandlers als Mikrofon für ein Cochlea-Implantat gezeigt. In dieser Ausfübrungsform besteht der Hohlkörper **8** aus einer flachen elliptischen Dose, die mit der einen Seite mit dem Gelenkknorpel **7** des durchtrennten Hammer-Amboß-Gelenks **5** und an ihrer anderen Seite mit einem in die Nische des ovalen Fensters **6** gestellten und im knöchernen Kanal der Stapediussehne fixierten Ständer **21** verbunden ist. Die Schwingungen des Trommelfells **1** werden von Hammer **2**, Amboß **3** und Gelenkknorpel **7** auf die dünne Schale des Hohlkörpers **8** übertragen. Durch den Kontakt über den Gelenkknorpel wird das Risiko einer Beeinträchtigung des Knochengewebes des Amboß **3** minimiert.

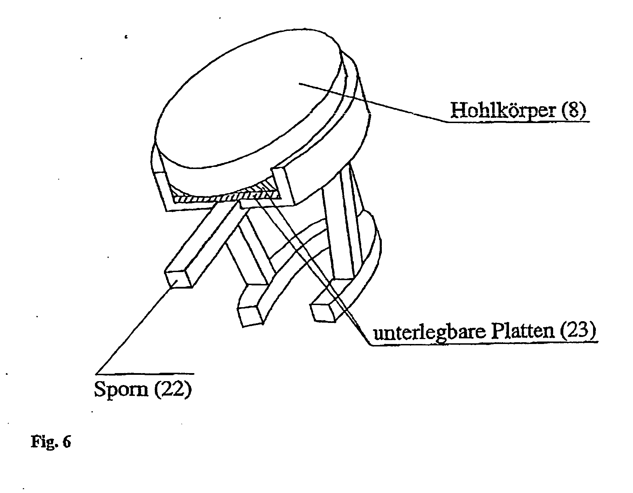

In der Fig. 6 ist die in Fig. 2 gezeigte Ausführungsform des Wandlers näher erläutert. Der Hohlkörper **8** besteht aus einer elliptischen plattenförmigen dünnen Schale und einem zu einem elliptischen Hohlzylinder mit Boden ausgeformten stabilen Rand. Der stabile Rand des Hohlkörpers **8** ist über unterlegbare Plättchen **23** mit dem Ständer verbunden, wobei letzterer einen Sporn **22** als Mittel zur Verankerung im knöchernen Kanal der Stapediussehne aufweist.

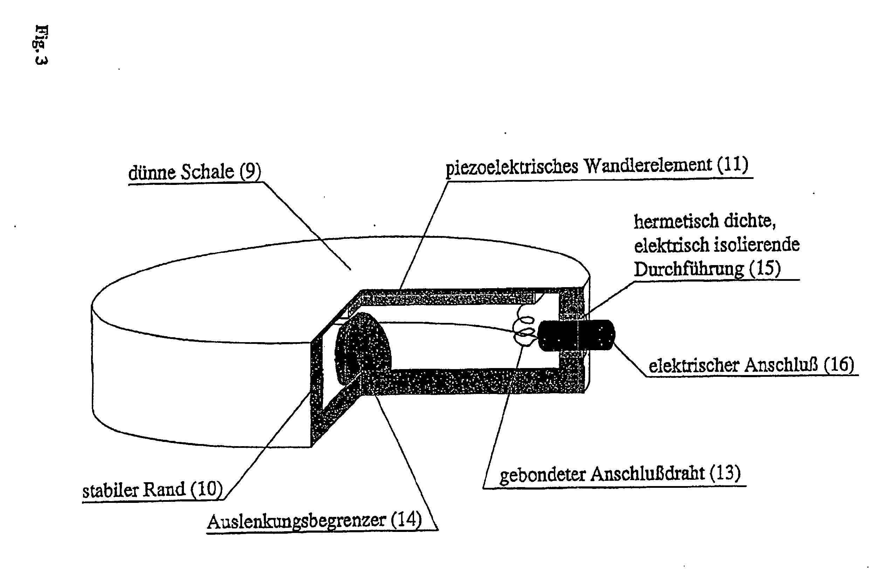

In der Fig. 3 ist eine Ausführungsform des Hohlkörpers sowie der durch ihn beherbergten Strukturen gezeigt. Hierbei besteht der Hohlkörper aus einer elliptischen plattenförmigen dünnen Schale **9** und einem zu einem elliptischen Hohlzylinder mit Boden ausgeformten stabilen Rand **10** aus Titan oder einer Titanlegierung. An die dünne Schale **9** ist ein plattenförmiges piezoelektrisches Wandlerelement **11** elektrisch leitend geklebt. Die elektrische Spannung bezüglich des Titanhohlkörpers wird durch einen gebondeten Anschlußdraht **13** zu einem elektrischen Anschluss **16** geführt, welcher über eine hermetisch dichte, elektrisch isolierende Durchführung **15** mit dem stabilen Rand **10** verbunden ist. Um zu hohe mechanische Belastungen der dünnen Schale **9** zu vermeiden, kann der Maximalausschlag durch einen Auslenkungsbegrenzer **14** begrenzt werden.

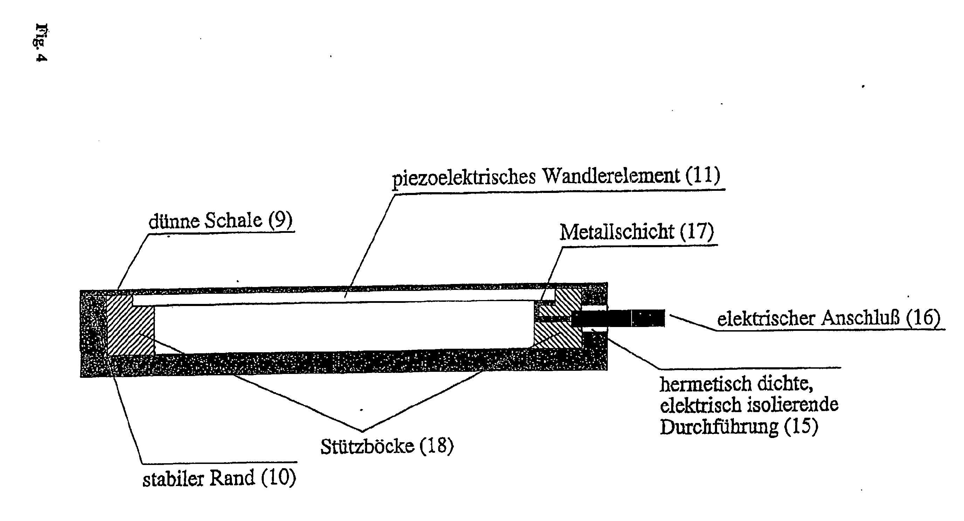

In der Fig. 4 ist eine weitere Ausführungsform des Hohlkörpers sowie der durch ihn beherbergten Strukturen gezeigt. Hierbei bestehen dünne Schale **9** und stabiler Rand **10** wiederum aus Titan oder einer Titanlegierung. Das piezoelektrische Wandlerelement **11** wird nun von Stützböcken **18** gehalten. Durch eine Metallschicht **17** auf einem oder beiden Stützböcken kann eine Kontaktierung des piezoelektrischen Wandlerelements auf einfache Weise realisiert werden. Die Metallschicht **17** ist mit dem elektrischen Anschluss **16** verbunden, wobei letzterer wieder über eine hermetisch dichte, elektrisch isolierende Durchführung **15** mit dem stabilen Rand **10** verbunden ist.

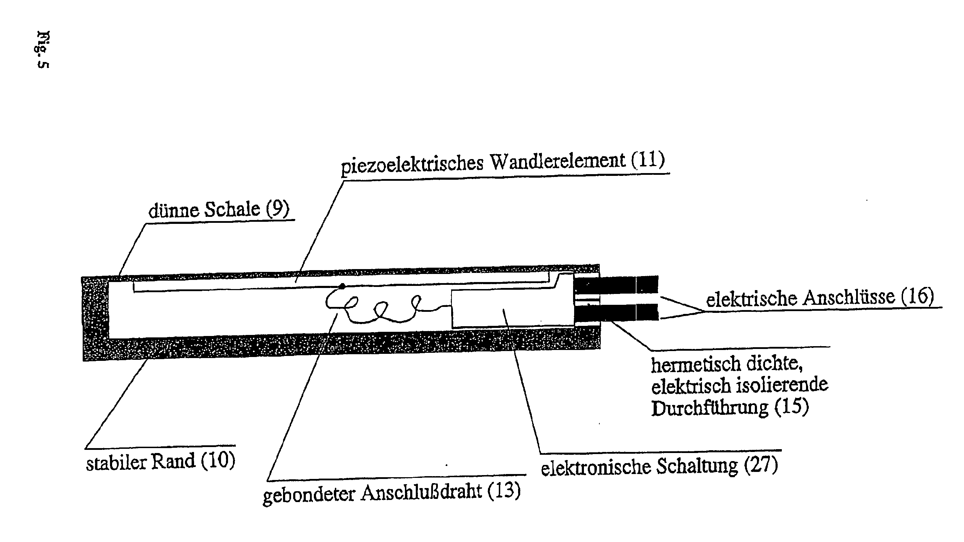

In der Fig. 5 ist eine weitere Ausführungsform des Hohlkörpers sowie der durch ihn beherbergten Strukturen gezeigt. Dünne Schale **9** und stabiler Rand **10** bestehen aus Titan oder einer Titanlegierung, an die dünne Schale **9** ist ein plattenförmiges piezoelektrisches Wandlerelement **11** elektrisch leitend geklebt. Die elektrische Spannung bezüglich des Titanhohlkörpers wird durch einen gebondeten Anschlußdraht **13** zu einer elektronischen Schaltung **27** geführt, die der Konditionierung der Signalspannung dient. Da die Schaltung **27** mit einer Betriebsspannung versorgt werden muss, werden in diesem Fall zwei elektrische Anschlüsse **16** vorgesehen, welche über hermetisch dichte, elektrisch isolierende Durchführungen **15** mit dem stabilen Rand **10** verbunden sind.

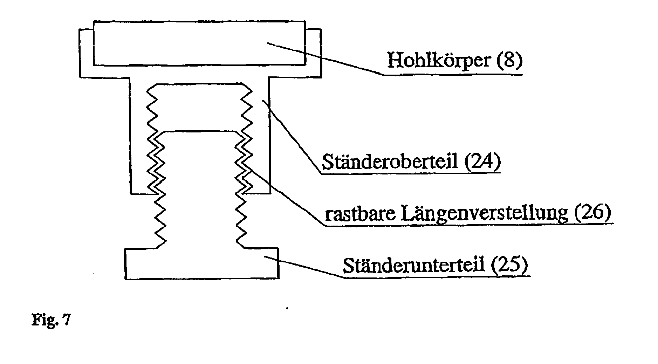

In der Fig. 7 ist eine Ausführungsform des Wandlers gezeigt, bei der das Gegenlager durch einen in der Nische des ovalen Fensters abstützbaren Ständer gebildet wird. Zur Positionierung des Hohlkörpers **8** besteht der Ständer aus einem Oberteil **24** und einem Unterteil **25,** welche über eine rastbare Längenverstellung **26** miteinander verbunden sind.

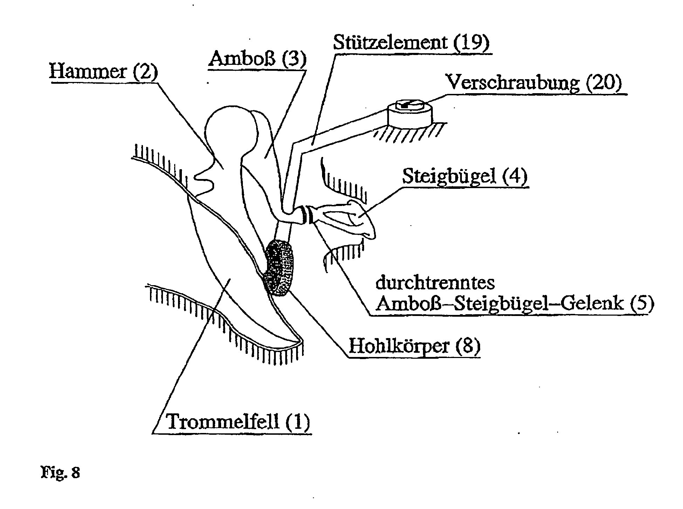

In der Fig. 8 ist ein Querschnitt des Mittelohrbereiches mit einer weiteren Ausführungsform des implantierten erfindungsgemäßen Wandlers als Mikrofon für ein implantierbares Hörgerät gezeigt. In dieser Ausführungsform besteht der Hohlkörper **8** wieder aus einer flachen elliptischen Dose, die an den Hammer **2** angekoppelt ist. Das Gegenlager wird hier durch ein mit dem stabilen Rand der Dose verbundenes Stützelement **19** realisiert, welches zu einer Verschraubung **20** mit einem Knochen des Mittelohrraumes führt. Zur Vermeidung einer Rückkopplung der Schwingungen der mit dem Steigbügel **4** in Verbindung stehenden Innenohrflüssigkeit auf den implantierten Wandler wird das Hammer-Amboß-Gelenk **5** durchtrennt. Die Schwingungen des Trommelfells **1** werden auf Hammer **2,** Amboß **3** und die dünne Schale des Hohlkörpers **8** übertragen.

### Bezugszeichenliste

- 1 -: Trommelfell
- 2 -: Hammer
- 3 -: Amboß
- 4 -: Steigbügel
- 5 -: Amboß-Steigbügel-Gelenk
- 6 -: ovales Fenster
- 7 -: Gelenkknorpel
- 8 -: Hohlkörper
- 9 -: Schale
- 10 -: Rand
- 11 -: Wandlerelemente
- 13 -: Anschlussdraht
- 14 -: Auslenkungsbegrenzer
- 15 -: Durchführung
- 16 -: Anschluss
- 17 -: Metallschicht
- 18 -: Stützbock
- 19 -: Stützelement
- 20 -: Verschraubung
- 21 -: Ständer
- 22 -: Sporn
- 23 -: Plättchen
- 24 -: Ständeroberteil
- 25 -: Ständerunterteil
- 26 -: Längenverstellung
- 27 -: Schaltung

## Patentansprüche

1. Implantierbarer elektromechanischer Wandler zum Empfangen von Schwingungen der Gehörknöcheichen und zur Umwandlung in eine elektrische Spannung, zum Einsatz als Mikrofon für ein Cochlea Implantat oder ein implantierbares Hörgerät, bestehend aus einem oder mehreren, von einem hermetisch dichten Hohlkörper (2) aus biokompatiblem Material umhausten, piezoelektrischen Wandlerelementen (11), wobei der Hohlkörper eine dünne Schale (9) aufweist, wobei die dünne Schale (9) von einem stabilen Rand (10) gehalten wird, wobei der stabile Rand an ein Gegenlager im Mittelohrraum ankoppelbar ist, **dadurch gekennzeichnet, dass** an der Innenseite des Hohlkörpers (8) die piezoelektrischen Wandlerelemente (11) mit der dünnen Schale (9) verbunden sind, und dass die Außenseite der dünnen Schale (9) derart zur flächigen Ankoppelung an ein Gehörknöchelchen vorgesehen ist, dass die Schwingungen der Gehörknöchelchen zu Verformungen der piezoelektischen Wandlerelemente (11) und zu einer elektrischen Spannung an Elektroden der Wandlerelemente (11) führen.

2. Wandler nach Anspruch 1, **dadurch gekennzeichnet, dass** der stabile Rand (10) die Form eines elliptischen Hohlzylinders aufweist.

3. Wandler nach Anspruch 1 oder 2, **dadurch gekennzeichnet, dass** der Hohlkörper (8) ein Mittel zur Begrenzung der Auslenkung der dünnen Schale aufweist.

4. Wandler nach einem der Ansprüche 1 bis 3, **dadurch gekennzeichnet, dass** das biokompatible Material des Hohlkörpers (8) Titan oder eine Titanlegierung ist.

5. Wandler nach einem der Ansprüche 1 bis 4, **dadurch gekennzeichnet, dass** die dünne Schale (9) als Platte mit einer Dicke von 20 bis 50 µm ausgebildet ist.

6. Wandler nach einem der Ansprüche 1 bis 5, **dadurch gekennzeichnet, dass** die Verbindung zwischen der dünnen Schale (9) und dem stabilen Rand (10) geschweißt ist.

7. Wandler nach einem der Ansprüche 1 bis 5, **dadurch gekennzeichnet, dass** dünne Schale (9) und stabiler Rand (10) aus einem Ganzen bestehen und die Formgebung durch ein mechanisches Trenn- oder Umformverfahren oder durch ein Ätzverfahren erreicht wird.

8. Wandler nach einem der Ansprüche 1 bis 7, **dadurch gekennzeichnet, dass** die piezoelektrischen Wandlerelemente (11) aus Piezokeramik, Piezofilm oder piezoelektrischen Einkristallen, vorzugsweise aus Bleizinkniobat-Bleititatnat (PZN-PT) oder Bleimagnesiumniobat-Bleititanat (PMN-PT), bestehen.

9. Wandler nach einem der Ansprüche 1 bis 8, **dadurch gekennzeichnet, dass** die piezoelektrischen Wandlerelemente (11) mit der dünnen Schale (9) verklebt sind.

10. Wandler nach einem der Ansprüche 1 bis 8, **dadurch gekennzeichnet, dass** die piezoelektrischen Wandlerelemente (11) in Kontakt zur dünnen Schale (9) mechanisch gelagert sind.

11. Wandler nach einem der Ansprüche 1 bis 10, **dadurch gekennzeichnet, dass** die piezoelektrischen Wandlerelemente (11) als uni- oder multimorfe Biegeplatten oder - balken ausgeführt sind.

12. Wandler nach einem der Ansprüche 1 bis 11, **dadurch gekennzeichnet, dass** eine elektronische Schaltung (27), welche sich im Inneren des Hohlkörpers (8) befindet, zur Konditionierung der an den elektrischen Wandlerelementen (11) abgegriffenen elektrischen Spannung vorgesehen ist.

13. Wandler nach einem der Ansprüche 1 bis 12, **dadurch gekennzeichnet, dass** zum externen Abgriff der an den piezoelektrischen Wandlerelementen (11) anliegenden und gegebenenfalls elektronisch konditionierten elektrischen Spannung hermetisch dichte, elektrisch isolierende Durchführungen (15) durch den stabilen Rand (10) vorgesehen sind.

14. Wandler nach Anspruch 13, **dadurch gekennzeichnet, dass** die hermetisch dichten, elektrisch isolierenden Durchführungen (15) aus Glas, Keramik oder Mineralien bestehen.

15. Wandler nach einem der Ansprüche 1 bis 14, **dadurch gekennzeichnet, dass** die dünne Schale (9) an den mit dem langen Amboßfortsatz verbundenen Gelenkknorpel (7) des durchtrennten Amboß-Steigbügel-Gelenks (5) an koppelbar ist.

16. Wandler nach einem der Ansprüche 1 bis 15, **dadurch gekennzeichnet, dass** das Gegenlager von einem Ständer (21) aus biokompatiblem Material gebildet wird, welcher an einem Ende in der Nische des ovalen Fensters (6) abstützbar und am anderen Ende zur Aufnahme des stabilen Randes (10) ausgebildet ist.

17. Wandler nach Anspruch 16, **dadurch gekennzeichnet, dass** der Ständer (21) ein Mittel zur Verankerung im knöchernen Kanal der Stapediussehne aufweist.

18. Wandler nach Anspruch 16 oder 17, **dadurch gekennzeichnet, dass** der Ständer (21) ein Mittel zur Positionierung der dünnen Schale (9) relativ zum anzukoppelnden Gehörknöchelchen aufweist.

19. Wandler nach Anspruch 18, **dadurch gekennzeichnet, dass** das Mittel zur Positionierung durch unterlegbare kleine Plättchen oder Keile aus biokompatiblern Material gebildet wird, welche zwischen Ständer (21) und stabilem Rand (10) eingebracht werden.

20. Wandler nach Anspruch 18, **dadurch gekennzeichnet, dass** das Mittel zur Positionierung als Zweiteilung des Ständers (21) ausgebildet ist, wobei beide Teile rastbar miteinander verbunden sind.

21. Wandler nach einem der Ansprüche 1 bis 15, **dadurch gekennzeichnet, dass** das Gegenlager durch ein an einem Ende mit dem stabilen Rand (10) verbundenen und am anderen Ende mit einem Knochen verschraubbaren Stützelement (19) gebildet wird.

## Claims

1. An implantable electromechanical transducer for receiving vibrations of the ossicles and for conversion into electrical voltage, for use as a microphone for a cochlear implant or an implantable hearing aid, consisting of one or several piezoelectric transducer elements (11) which are enclosed by a hermetically sealed hollow body (2) made of biocompatible material, with the hollow body comprising a thin shell (9), with the thin shell (9) being held by a stable edge (10), wherein said stable edge can be coupled with a counter-bearing in the middle ear space, **characterized in that** the piezoelectric transducer elements (11) are connected with the thin shell (9) on the inside of the hollow body (8), and that the outside of the thin shell (9) is provided for flat coupling to an ossicle in such a way that the vibrations of the ossicles lead to deformations of the piezoelectric transducer elements (11) and to an electric voltage on electrodes of the transducer elements (11).

2. A transducer according to claim 1, **characterized in that** the stable edge (10) has the shape of an elliptical hollow cylinder.

3. A transducer according to claim 1 or 2, **characterized in that** the hollow body (8) comprises a means for limiting the deflection of the thin shell.

4. A transducer according to one of the claims 1 to 3, **characterized in that** the biocompatible material of the hollow body (8) is titanium or a titanium alloy.

5. A transducer according to one of the claims 1 to 4, **characterized in that** the thin shell (9) is arranged as a plate with a thickness of 20 to 50 µm.

6. A transducer according to one of the claims 1 to 5, **characterised in that** the connection between the thin shell (9) and the stable edge (10) is welded.

7. A transducer according to one of the claims 1 to 5, **characterized in that** the thin shell (9) and the stable edge (10) are integral and the shaping is achieved by a mechanical separation or forming process, or by an etching process.

8. A transducer according to one of the claims 1 to 7, **characterized in that** the piezoelectric transducer elements (11) consist of piezoceramics, piezofilm or piezoelectric monocrystals, preferably of lead zinc niobate-lead titanate (PZN-PT) or lead magnesium niobate-lead titanate (PMN-PT).

9. A transducer according to one of the claims 1 to 8, **characterized in that** the piezoelectric transducer elements (11) are glued together with the thin shell (8).

10. A transducer according to one of the claims 1 to 8, **characterized in that** the piezoelectric transducer elements (11) in contact with the thin shell (9) arc mechanically mounted.

11. A transducer according to one of the claims 1 to 10, **characterized in that** the piezoelectric transducer elements (11) are arranged as monomorphous or multimorphous bending plates or beams.

12. A transducer according to one of the claims 1 to 11, **characterized in that** an electronic circuit (27) which is disposed in the interior of the hollow body (8) is provided for conditioning the electrical voltage tapped from the electrical transducer elements (11).

13. A transducer according to one of the claims 1 to 12, **characterized in that** hermetically sealed, electrically insulating passages (15) are provided through the stable edge (10) for externally tapping the electrical voltage which is impressed on the piezoelectric transducer elements (11) and is optionally electronically conditioned.

14. A transducer according to one of the claims 1 to 13, **characterized in that** the hermetically sealed, electrically insulating passages (15) consist of glass, ceramics or minerals.

15. A transducer according to one of the claims 1 to 14, **characterized in that** the thin shell (9) can be coupled to the articular cartilage (7) of the severed incudostapedial joint (5), said articular cartilage connected with the long protrusion of the anvil.

16. A transducer according to one of the claims I to 15, **characterized in that** the counter-bearing is formed by a post (21) made of biocompatible material, which can be supported at one end in the recess of the oval window (6) and is arranged at the other end for receiving the stable edge (10).

17. A transducer according to claim 16, **characterized in that** the post (21) comprises a means for anchoring in the osseus canal of the stapedius tendon.

18. A transducer according to claim 16 or 17, **characterized in that** the post (21) comprises a means for positioning the thin shell (9) relative to the ossicle to be coupled.

19. A transducer according to claim 18, **characterized in that** the means for positioning is formed by small platelets or wedges made of biocompatible material which are introduced between the post (21) and the stable edge (10) and placed underneath.

20. A transducer according to claim 18, **characterized in that** the means for positioning is arranged as a division of the post (21), with both parts being latchably connected with each other.

21. A transducer according to one of the claims 1 to 15, **characterized in that** the counter-bearing is formed by a support element (19) which is connected at one end with the stable edge (10) and can be screwed together at the other end with a bone.

## Revendications

1. Convertisseur électromécanique implantable destiné à capter des vibrations des osselets de l'ouïe et à les convertir en une tension électrique, utilisable comme microphone pour un implant cochléaire ou un appareil auditif implantable, composé d'un ou plusieurs éléments convertisseurs piézoélectriques (11) logés dans un corps creux (2) hermétique en matériau biocompatible, lequel corps creux présente une coque mince (9), la coque mince (9) étant maintenue par un bord stable (10), lequel bord stable peut être couplé à un contre-appui dans l'espace de l'oreille moyenne, **caractérisé en ce que** les éléments convertisseurs piézoélectriques (11) sont reliés à la coque mince (9) sur la face interne du corps creux (8) et **en ce que** la face externe de la coque mince (9) est prévue pour se coupler en surface sur un osselet de l'ouïe de telle manière que les vibrations des osselets de l'ouïe produisent des déformations des éléments convertisseurs piézoélectriques (11) et une tension électrique sur des électrodes des éléments convertisseurs (11).

2. Convertisseur selon la revendication 1, **caractérisé en ce que** le bord stable (10) présente la forme d'un cylindre creux elliptique.

3. Convertisseur selon la revendication 1 ou 2, **caractérisé en ce que** le corps creux (8) présente un moyen pour limiter la déviation de la coque mince.

4. Convertisseur selon l'une des revendications 1 à 3, **caractérisé en ce que** le matériau biocompatible du corps creux (8) est du titane ou un alliage de titane.

5. Convertisseur selon l'une des revendications 1 à 4, **caractérisé en ce que** la coque mince (9) est conformée comme une plaque de 20 à 50 µm d'épaisseur.

6. Convertisseur selon l'une des revendications 1 à 5, **caractérisé en ce que** la liaison entre la coque mince (9) et le bord stable (10) est soudée.

7. Convertisseur selon l'une des revendications 1 à 5, **caractérisé en ce que** la coque mince (9) et le bord stable (10) forment un tout et la mise en forme est obtenue par un procédé de découpe ou de formage mécanique ou par un procédé d'attaque chimique.

8. Convertisseur selon l'une des revendications 1 à 7, **caractérisé en ce que** les éléments convertisseurs piézoélectriques (11) se composent de piézocéramique, de piézofilm ou de monocristaux piézoélectriques, de préférence en niobate de zinc et de plomb et titanate de plomb (PZN-PT) ou en niobale de plomb et de magnésium et titanate de plomb (PMN-PT).

9. Convertisseur selon l'une des revendications 1 à 8, **caractérisé en ce que** les éléments convertisseurs piézoélectriques (11) sont collés à la coque mince (9).

10. Convertisseur selon l'une des revendications 1 à 8, **caractérisé en ce que** les éléments convertisseurs piézoélectriques (11) sont supportés mécaniquement au contact de la coque mince (9).

11. Convertisseur sclon l'une des revendications 1 à 10, **caractérisé en ce que** les éléments convertisseurs piézoélectriques (11) sont réalisés sous la forme de plaques ou poutres flexibles mono- ou multimorphes.

12. Convertisseur selon l'une des revendications 1 à 11, **caractérisé en ce qu'**un circuit électronique (27) disposé à l'intérieur du corps creux (8) est prévu pour conditionner la tension électrique captée au niveau des éléments convertisseurs piézoélectriques (11).

13. Convertisseur selon l'une des revendications 1 à 12, **caractérisé en ce qu'**il est prévu pour l'acquisition externe de la tension électrique présente au niveau des éléments convertisseurs piézoélectriques (11) et éventuellement conditionnée des traversées (15) hermétiques et électriquement isolantes à travers le bord stable (10).

14. Convertisseur selon la revendication 13, **caractérisé en ce que** les traversées (15) hermétique et électriquement isolantes (15) sont faites de verre, de céramique ou de matières minérales.

15. Convertisseur selon l'une des revendications 1 à 14, **caractérisé en ce que** la coque mince (9) peut être couplée au cartilage articulaire (7) de l'articulation incudo-stapédienne (5) sectionnée relié à la longue apophyse de l'enclume.

16. Convertisseur selon l'une des revendications 1 à 15, **caractérisé en ce que** le contre-appui est formé par un support (21) en matériau biocompatible qui peut s'appuyer à une extrémité dans la niche de la fenêtre ovale (6) et qui est forme à l'autre extrémité de façon à recevoir le bord stable (10).

17. Convertisseur selon la revendication 16, **caractérisé en ce que** le support (21) présente un moyen d'ancrage dans le canal osseux du tendon du muscle stapédien.

18. Convertisseur selon la revendication 16 ou 17, **caractérisé en ce que** le support (21) présente un moyen de positionnement de la coque mince (9) par rapport aux osselets de l'ouïe à coupler.

19. Convertisseur selon la revendication 18, **caractérisé en ce que** les moyens de positionnement sont formés par des plaquettes ou des coins en matériau biocompatible pouvant être intercalés, qui sont insérés entre le support (21) et le bord stable (10).

20. Convertisseur selon la revendication 18, **caractérisé en ce que** le moyen de positionnement est réalisé comme une division en deux du support (21), dont les deux parties sont reliées entre elles avec possibilité d'emboîtement.

21. Convertisseur selon l'une des revendications 1 à 15, **caractérisé en ce que** le contre-appui est formé par un élément d'appui (19) relié à une extrémité au bord stable (10) et pouvant être vissé à un os à l'autre extrémité.
